(19) Europäisches Patentamt
European Patent Office
Office européen des brevets

(11) EP 2 433 624 B1

(12) **EUROPEAN PATENT SPECIFICATION**

(45) Date of publication and mention of the grant of the patent:
**21.08.2013 Bulletin 2013/34**

(51) Int Cl.:
***A61K 31/122*** *(2006.01)* ***A61P 33/00*** *(2006.01)*
***A01N 35/06*** *(2006.01)* ***A61K 9/14*** *(2006.01)*

(21) Application number: **10394021.9**

(22) Date of filing: **23.09.2010**

(54) **Use of buparvaquone for controlling mites parasitising honeybees**

Verwendung von Buparvaquone zur Bekämpfung von Bienen parasitierende Milben

Utilisation du buparvaquone pour contrôler les acariens parasitisants les abeilles

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**28.03.2012 Bulletin 2012/13**

(73) Proprietor: **Bimeda International Pharmaceuticals Limited**
**Dublin 24 (IE)**

(72) Inventors:
- **McHardy, Nicholas**
  **Blessington**
  **County Wicklow (IE)**
- **Smith, Ronan**
  **Dublin 14 (IE)**
- **Brady, Paul Declan**
  **Bray**
  **County Wicklow (IE)**

(74) Representative: **O'Brien, John Augustine et al**
**John A. O'Brien & Associates**
**Third Floor,**
**Duncairn House,**
**14 Carysfort Avenue**
**Blackrock, Co Dublin (IE)**

(56) References cited:
- **MBWAMBO H A ET AL: "Evaluation of buparvaquone (BUTA-Kel(TM) KELA, Belgium) as a treatment of East Coast fever in cattle, in the peri-urban of Dar Es Salaam city, Tanzania", VETERINARY PARASITOLOGY, ELSEVIER SCIENCE, AMSTERDAM, NL, vol. 139, no. 1-3, 30 June 2006 (2006-06-30), pages 67-73, XP025025702, ISSN: 0304-4017, DOI: DOI: 10.1016/J.VETPAR.2006.02.024 [retrieved on 2006-06-30]**
- **KAYSER O ET AL: "Formulation and biopharmaceutical issues in the development of drug delivery systems for antiparasitic drugs.", PARASITOLOGY RESEARCH, vol. 90, no. Supplement 2, June 2003 (2003-06), pages S63-S70, XP002621487, ISSN: 0932-0113**
- **KAYSER O: "A new approach for targeting to Cryptosporidium parvum using mucoadhesive nanosuspensions: Research and applications", INTERNATIONAL JOURNAL OF PHARMACEUTICS (KIDLINGTON), vol. 214, no. 1-2, 19 February 2001 (2001-02-19), pages 83-85, XP002621488, ISSN: 0378-5173**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 433 624 B1

## Description

Introduction

The invention relates to controlling *Varroa* mites that parasitise honey bees. *Varroa* mites are a severe health hazard to bees, killing or debilitating them, frequently causing the demise of the whole colony. This has a serious impact on honey production and the pollinating capacity of bees.

*Varroa* mites also transmit various viral, bacterial and fungal infections to bees. Thus, control of the mites can produce indirect benefits to the health and productivity of the bees.

Opinion is divided on whether *Varroa* mites are directly or indirectly associated with the 'colony collapse syndrome' of bees that currently is causing such concern wordwide. The cause of this syndrome remains unclear but it is generally accepted that additional infestation by *Varroa* mites could only intensify the problem.

Various licenced products that claim to be effective in controlling *Varroa* mites are available to both commercial and "hobby" beekeepers, but none is particularly effective.

Known licensed products, which include pyrethroids and organphosphates, have well known insecticidal properties so they have to be used carefully to avoid harming the bees. Unlicenced products, such as formic acid, appear to have only modest miticidal effects and unknown effects on bees.

Known miticides are applied to bees within the hive in a number of different ways. The methods include sprays, smokes, aerosols, dusts, vaporisers, brushes, etc to apply them to the bees and 'brood combs' and thus to the mites that parasitise them. They are applied routinely as preventive treatments, mostly in the spring and autumn when the bees are confined to the hives. This also reduces the risk of contamination of honey and beeswax. The aim is to minimise the infestation of bee larvae, which are particularly susceptible to such parasites. Once attached, the mites commonly remain on the bees for life. They feed on the haemolymph (blood) of the bees, hence their debilitating and lethal effects. Several mites may attach to each bee so the weight of mites may exceed 10% of the weight of the uninfested bee.

In the face of overt infestations, which are frequent, given the relatively low efficacy of existing products, miticides are often applied therapeutically, regardless of the time of year, with greatly increased risk of contamination of honey and wax.

Mbwambo et al (Veterinary Parasitology (2006) Vol. 139: no. 1-3; p 67-73) evaluated the efficacy of buparvaquone as a treatment of East Coast fever (*Theileria parva* infection) in cattle in the peri-urban of Dar Es Salaam city, Tanzinia.

The review of Kayser et al (Parasitology Research (2003) Vol. 90: no. supplement 2; p s63-s70) discusses formulation and biopharmaceutical issues in the development of drug delivery systems for antiparasitic drugs, in particular the strategy of optimal formulation and application for antiparasitic drugs.

Kayser (International Journal of Pharmaceutics (2001) Vol. 214, p 83-85) presents a new strategy to deliver antibiotics to the *cryptosporidium* - infected gastrointestinal tract of patients using a mucoadhesive nanosuspension containing bupravaquone and chitosan.

Summary of the Invention

According to the invention there is provided a method for controlling mites (especially *Varroa* mites) parasitising honeybees, which comprises administering to the honeybees a non-toxic miticidally effective amount of burparvaquone.

In one embodiment said administering comprises feeding buparvaquone to the bees. Said administering may comprise feeding buparvaquone in a concentrated sugar solution to the bees.

In another aspect the invention provides a composition for controlling mites parasitising honeybees comprising a commonly used diet supplement for honeybees which contains a miticidal amount of buparvaquone that is entirely non-toxic for bees and bee larvae.

The bee diet may comprise an aqueous solution of a sugar, most commonly sucrose, at a high concentration, commonly around 66%. The diet may also contain other nutrients and other supplements such as minerals, vitamins and pharmaceutical substances, for example bactericides and fungicides.

The composition may comprise a carrier for buparvaquone. The carrier may comprise a solvent such as N-methyl-pyrollidone (NMP).

We have found that buparvaquone is highly effective in killing the mites while being entirely safe for bees, both larvae and adults.

Buparvaquone can be applied either as a spray or by inclusion in the concentrated sugar solution that is fed to bees throughout the active season of the bees. As the bees ingest the medicated feed, they take up the buparvaquone, which then crosses from the gut lumen into the haemolymph. The mites ingest the buparvaquone and are affected by it, as they suck the haemolymph. Because buparvaquone is not present on the surface of the bees, as it would be if it was applied as a topical treatment such as a dust, and it is not present in a form such as an aerosol that would pervade the whole hive, the chances of it contaminating honey and wax are minimised. Furthermore, any buparvaquone that is not absorbed from the digestive tract of the bee and which is voided in the faeces will not contaminate honey or wax because bees do not defecate on either the honey-bearing combs or brood combs, but elsewhere within or outside the hive.

Affected mites, following treatment with buparvaquone, quickly detach from the bees and larvae. They may then survive for a few hours but they appear to be paralysed and are unable to re-attach. This is in marked contrast to known products. Though such known products may kill some mites, those that detach and survive may recover and re-attach. The effect of some products, apparently, is solely to cause detachment but not death of the mites. These mites are readily able to re-attach, particularly if detachment occurs within the hive, and most importantly, if it occurs on or around the brood combs.

We have found that buparvaquone is highly effective for the prevention and treatment of *Varroa* mite infesations of bees, whilst being entirely without adverse effect on the bees themselves. It can be administered safely and conveniently simply by incorporation in bee diet. Furthermore, the risk of contamination of honey and beeswax with product residues is minimal and, in any case, the buparvaquone is of extremely low toxicity to mammals. Thus, it presents no hazard to beekeepers or consumers of honey or beeswax. In addition, because buparvaquone has no insecticidal or other toxic properties, its environmental effect on insects and other animals that may come into contact with it is negligible.

## Brief Description of the Figures

The invention will be more clearly understood from the following description thereof, given by way of example only, in which:

Fig. 1 percentage mortality of bees at different concentrations of the BPQ A/ solvent over a 16 day test period;

Fig. 2 bee mortality per cage at each concentration of product over time;

Fig. 3 bee mortality per cage at different concentrations of solvent over time;

Fig. 4 mean bee mortality at different treatment concentrations over time;

Fig. 5 mean been mortality at different solvent concentrations over time;

Fig. 6 the mean volume of product and solvent treated food consumed by cage bees over the 16 day treatment period;

Fig. 7 the percentage survival of caged bees sprayed with different concentrations of BPQ A and solvent over a 7 day period;

Fig. 8 the mean number of bees surviving when sprayed with an aqueous solution of product or solvent over a 7 day period;

Fig. 9 mean survival rate of *Varroa* mites over time when treated with different concentrations of BPQ;

Fig. 10 the mean percentage efficacy of BPQ fed in a sucrose solution at different concentrations;

Fig. 11 the percentage efficacy of BPQ fed in a sucrose solution at different concentrations in each of the replicates;

Fig. 12 the number of bees surviving on day 5 in each of the replicates at different concentrations;

Fig. 13 the mean percentage efficacy of BPQ sprayed as an aqueous solution; and

Fig. 14 the percentage efficacy of the BPQ against the *Varroa* mite when sprayed on adult bees as an aqueous solution.

## Detailed Description

The active ingredient in the invention is buparvaquone (BPQ), a hydroxynaphthoqune compound that has been used for the treatment of certain tick-transmitted diseases of farm livestock.

BPQ may be supplied already formulated into sugar-based bee diet or as a solution in an organic solvent such as N-methyl-pyrrolidone (NMP) or dimethylsulphoxide (DMSO) for incorporation into bee diet by diet suppliers or bee owners.

Bee diet is typically a 66% aqueous solution of sucrose. Commercially-supplied bee diets may contain a number of other additives, including minerals, vitamins, other medicaments etc. Buparvaquone is extremely stable and non-reactive, and is therefore unlikely to react in any way with any other additives in the diet.

BPQ is completely insoluble in water and it immediately falls out of solution in organic solvents on contact with water. If applied to *Varroa* mites in its solid form it has no miticidal effect because the particles are too large to pass across the cuticle of the mites. Similarly, if fed to bees in its solid form, it is not absorbed into the haemolymph in sufficient amounts to kill mites that ingest the bee's haemolymph.

Preferably the buparvaquone is presented as a solution in, for example, NMP because in this form, when used as a spray, it readily crosses the mite's cuticle or if ingested by the bee it would enter the haemolymph readily. However, an external spray rapidly interferes with the bee's ability to fly. Similarly, there are problems in incorporating BPQ directly into bee diet because the BPQ precipitates from solution, and may form a sticky mass, on contact with the water in the diet. In any case, bees are very fastidious eaters and they refuse to consume diet that contains significant amounts of volatile substances such as NMP or DMSO.

Therefore, the BPQ composition of the invention is preferably supplied as an aqueous suspension of extremely finely-divided particles of BPQ, or a nano-suspension of BPQ which behaves almost as a solution. Particles of sufficient fineness can be prepared in several ways, commonly used in pharmaceutical formulations, such as micronisation. However, the nature of BPQ lends itself to a simpler and less expensive process, which is preferred. The BPQ is first dissolved in a solvent such as NMP or DMSO and this is sprayed into water. On contact with the water, the BPQ immediately precipitates as fine particles. The fineness of the particles can be varied in at least three ways:

concentration of BPQ in the solvent;
rate of addition of solution of BPQ to the water; and/or
aggitation of the water by e.g. mechanical or ultrasonic means.

The finest particles can be generally produced by spraying a dilute solution of BPQ into rapidly agitated water. Coalescence of these ultra-fine particles into larger particles can be prevented by known methods.

Efficacy and safety studies conducted to date have used aqueous suspensions of BPQ produced crudely in this way. Refinement and standardisation of the production method may significantly improve product performance by minimising particle size, thereby increasing its concentration in haemolymph when fed to bees, or by maximising the amount of BPQ absorbed from the bee's intestine into the haemolymph thereby minimising the amount of BPQ that must be ingested by the bees, or by increasing penetration of mite cuticle or entry into respiratory spiracles when applied as a spray.

The mode of BPQ's miticidal effect is not known. When used as a treatment for tick-transmitted protozoal diseses of livestock, its action is as an electron transport inhibitor but the electron transport systems of protozoa and mites are very dissimilar. Mites that detach from bees following treatment with BPQ become paralysed before death. This might suggest a neurological effect (in common with that of other varroacides such as pyrethroids and organophosphates). However, while the neurotransmitters of mites and insects are similar (hence pyrethroids and organophosphates are also insecticidal) BPQ has no insecticidal effect. The miticidal action of BPQ, therefore, may be unique, but its nature remains unknown. Furthermore, the toxicology of BPQ to mammals has revealed no neurotoxic effect whatsoever, even at dosages many thousands of times higher than those in the present invention.

The active ingredient is buparvaquone, (BPQ), supplied as a formulated product (BPQ **A**) and as a powder (BPQ **B**).

We have found that:

- Bees feed readily on sugar solutions containing BPQ
- A dose/toxicity relationship was found on bees fed the formulated BPQ A product (liquid diluted in sugar:water) over a 16 day period. Some of the bees fed this formulated product and groups fed solvent in sugar solution developed a sticky residue on their bodies. However, at the concentrations subsequently found to be effective in killing *Varroa* mites attached to bees, no significant toxicity to the bees was found to result from these deposits
- BPQ was found to be non-toxic to bee larvae.
- A modified formulation with dissolution of powdered BPQ **B,** in NMP was used with dilutions with water and then further dilution in sugar solution. This formulation was tested over 5days. No toxicity for bees or bee larvae was found at any concentration. No sticky residue was noted on bees.
- The formulation was >80% effective in Varroa knockdown at an extremely low concentration (0.0005$\mu$g/mL)
- Delivery of BPQ to mites via feeding of the agent to their bee hosts was found to be more effective than spraying. This result is important as administration through bee feed would be logistically much more attractive for bee keepers.

The high efficacy achieved at such low concentrations is remarkable. Toxicity for bees and bee larvae seems also to be low or absent even at concentrations much higher than the likely therapeutic dose.

BPQ was presented in two forms:

(a) a liquid at a concentration of 50mg/mL, (BPQ **A),** and

(b) a yellow powder version of BPQ (PBQ **B)** which can be dissolved in the solvent (N-methyl pyrollidone) and then diluted into aqueous medium.

We have assessed the toxicity of BPQ to adult honeybees (*Apis mellifera*) using the 50 mg/mL liquid solution, BPQ **A**, under laboratory conditions

**Example 1 - <u>Toxicity of BPQ A to honeybees when fed in a sugar syrup solution</u>** (50mL liquid at a concentration of 50mg BPQ/mL)

<u>Materials and methods</u>

<u>Hoarding cages:</u>

Hoarding cages consisted of plastic tumblers (600mL) with an opening diameter of 84mm. The tumblers were paired with standard plastic petri-dishes and glass scintillation vials. To allow sufficient ventilation, 33 holes, approximately 3mm in diameter were made in the sides of each tumbler, in addition to two 30mm x 30mm holes fitted with nylon mesh. A circular hole was cut out of the bottom of the cup, which produced an opening large enough to allow a lidded scintillation vial to pass through the hole. Two holes were drilled into the lids of the scintillation vials with a 1mm bit and these vials were filled a 1:1 sugar solution, together with different concentrations of the solvent and product. A piece of disposable incontinence underpad was cut to be slightly larger than the petri-dish so that drips from the scintillation vial would be absorbed.

<u>Honeybees</u>

A total of 30 newly emerged bees (1 day old) were placed in each of the 33 cages.

<u>Treatments</u>

Three replicates were analysed at each concentration of BPQ **A** and solvent as outlined below, together with a control group which were only fed sugar and water throughout the experimental period.

<u>Concentrations of BPQ and solvent in sugar:water feed</u>

<u>BP **A** in sugar:water feed</u>

0.5μg/mL. (C1-C3)
0.05μg/mL. (D1-D3)
0.005μg/mL. (E1-E3)
0.0005μg/mL. (F1-F3)
0.00005μg/mL (G1-G3)

<u>Solvent in sugar:water feed</u> (equivalent solvent concentration to that in BPQ **A**-sugar/water solutions)

0.001% (C4-C6)
0.0001%. (D4-D6)
0.00001%. (E4-E6)
0.000001%. (F4-F6)
0.0000001 (G4-G6)

Sugar:water feed only

X1-X3

Bees were maintained for 16 days at 32°C. Bees were fed additional food as required throughout the experimental period and the general behaviour and survival of the test bees were recorded on a daily basis. Solvent and control bees required additional food (treatment) on days 5 and day 12, while product treated bees took the food slightly slower and required feeding on days 7 and 13. On day 16 the experiment was completed.

Results

In product treated colonies the percentage mortality of bees ranged from 28% to 14%. In the solvent treated colonies, variation was much greater ranging from 27% to 3%, while the mean mortality for the untreated control group was 14% (Fig. 1).

In the BPQ **A** treated groups peaks in bee mortality were recorded on Day 8 and Day 14 (Fig. 2, 4), which was the day after administering the product. This was not as apparent in the solvent treated group, but mortality did increase over time reaching a peak on Day 11-14 (Fig. 3, 5). Mortality also increased in the untreated control group at the end of the treatment period. Caged bees consumed the solvent and syrup (control diet) at a slightly faster rate than the caged bees fed BPQ **A,** consequently required feeding on Day 5, Day 12 and Day 16. BPQ **A** treated bees were fed on Days 7, 13 and 16. The total consumption of food is given in Fig. 6.

**Example 2 - Toxicity of BPQ A in an aqueous solution when sprayed onto honeybees**

(50mL liquid at a concentration of 50mg/mL)

Material and methods

Hoarding cages were prepared as described in example 1. BPQ **A** and solvent were made up at similar concentrations as described in example 1, but in an aqueous solution, rather than a sugar /syrup solution. The latter was used to prevent bees becoming sticky when the solution is sprayed directly onto bees. Three replicates were analysed at each concentration of BPQ **A** and solvent, together with a control group which were only sprayed with water. Bees in all cages were initially fed with 10mL of sugar:water syrup using a gravity feeder and placed in an incubator at 32°C. Bees were fed further syrup when required and the percentage survival of bees in each treatment group were monitored on a daily basis over a 7 day period.

Concentrations of BPQ **A** and solvent in an aqueous (water) solution.

BPO **A** in aqueous solution

0.5μg/mL. (C1-C3)
0.05μg/mL. (D1-D3)
0.005μg/mL. (E1-E3)
0.0005μg/mL. (F1-F3)
0.00005μg/mL (G1-G3)

Solvent in sugar:water feed (equivalent solvent concentration to that in BPQ **A** -sugar/water solutions)

0.001% (C4-C6)
0.0001%. (D4-D6)
0.00001%. (E4-E6)
0.000001%. (F4-F6)
0.0000001 (G4-G6)

Results

The present cage experiment indicates that spraying the bees with BPQ **A** in an aqueous solution at the stated concentrations has no negative effects on survival rate (Figs. 7 and 8) or feeding behaviour of honeybees.

**Example 3 - Toxicity of BPQ A in an aqueous solution when sprayed on honeybee larvae** (50mL liquid at a concentration of 50mg/mL - BPQ A)

Aim

To spray eggs/larvae with different concentrations of the active ingredient/solvent and examine the effect on development of the young larvae

Materials and methods

Eggs/larvae of similar age were attained by placing the queen bee in a frame cage for 36 hrs. Once the frame was laid up, the queen was released and the frame was replaced in the colony for 3 days. The frame cage allowed worker bees to attend the larvae, but prevented the queen from laying in the frame. On day 3/4, when the eggs were changing to larvae, the frame was removed and divided temporarily in 6 x 5cm strips at either side using grease proof paper to facilitate the assessment of product and solvent. Within each strip, three 3.5cm x 2.5cm squares (approximately 30 cells) of grease proof paper was removed and the developing larvae sprayed with BPQ A/solvent at the concentrations described above.

Results

Results indicate that spraying BPQ **A** or solvent had no negative effects on honeybee larvae survival. The mean survival rate (±se) for the BPQ **A,** the solvent and the control were 86.1±1.19, 87.7±1.02 and 86.0±3.55 respectively.

Table 1

The percentage survival of honeybee larvae when sprayed with different concentrations of BPQ **A** and solvent. Table indicates three replicates for each treatment. Product = (c1-c3 - g1-g3) : Solvent c4-c6 - g4-g6)

| **Product** | **Total cells** | **Missing cells** | **Sealed cells** | **Young bees emerged** | **% Emergence** |
|---|---|---|---|---|---|
| c1 | 30 | 1 | 28 | 25 | 86.2 |
| c2 | 30 | 1 | 27 | 26 | 89.7 |
| c3 | 30 | 2 | 28 | 26 | 92.9 |
| d1 | 30 | 1 | 26 | 25 | 86.2 |
| d2 | 30 | 1 | 28 | 25 | 86.2 |
| d3 | 30 | 1 | 27 | 24 | 82.8 |
| e1 | 30 | 0 | 29 | 26 | 86.7 |
| e2 | 30 | 0 | 28 | 24 | 80.0 |
| e3 | 30 | 2 | 27 | 26 | 92.9 |
| f1 | 30 | 1 | 28 | 26 | 89.7 |
| f2 | 30 | 3 | 26 | 24 | 88.9 |
| f3 | 30 | 1 | 28 | 25 | 86.2 |
| g1 | 30 | 1 | 26 | 25 | 86.2 |
| g2 | 30 | 0 | 28 | 24 | 80.0 |
| g3 | 30 | 0 | 29 | 23 | 76.7 |
| **Solvent** | | | | | |
| c4 | 30 | 2 | 28 | 24 | 85.7 |
| c5 | 30 | 1 | 29 | 25 | 86.2 |
| c6 | 30 | 1 | 27 | 23 | 79.3 |
| d4 | 30 | 2 | 27 | 25 | 89.3 |

(continued)

| The percentage survival of honeybee larvae when sprayed with different concentrations of BPQ **A** and solvent. Table indicates three replicates for each treatment. Product = (c1-c3 - g1-g3) : Solvent c4-c6 - g4-g6) | | | | | |
|---|---|---|---|---|---|
| **Product** | **Total cells** | **Missing cells** | **Sealed cells** | **Young bees emerged** | **% Emergence** |
| d5 | 30 | 1 | 28 | 26 | 89.7 |
| d6 | 30 | 1 | 29 | 25 | 86.2 |
| e4 | 30 | 1 | 29 | 27 | 93.1 |
| e5 | 30 | 0 | 30 | 29 | 96.7 |
| e6 | 30 | 2 | 28 | 24 | 85.7 |
| f4 | 30 | 3 | 27 | 23 | 85.2 |
| f5 | 30 | 1 | 28 | 25 | 86.2 |
| f6 | 30 | 1 | 28 | 25 | 86.2 |
| g4 | 30 | 1 | 29 | 26 | 89.7 |
| g5 | 30 | 1 | 28 | 26 | 89.7 |
| g6 | 30 | 1 | 27 | 25 | 86.2 |
| **Control** | | | | | |
| x1 | 30 | 1 | 28 | 24 | 82.8 |
| x2 | 30 | 2 | 27 | 23 | 82.1 |
| x3 | 30 | 1 | 29 | 27 | 93.1 |

**Example 4 - <u>Toxicity of BPQ B to honeybee larvae when fed treated syrup</u>** (Powder version of BPQ, BPQ B, dissolved in the solvent (N-methyl pyrollidone)

<u>Aim</u>

To measure the effect of treatment on developing larvae when fed syrup treated with BPQ powder, BPQ **B,** dissolved in the solvent (N-methyl pyrollidone) and diluted in an aqueous solution.

<u>Materials and methods</u>

Apidae boxes were used to raise young queens and when virgin queens started to lay an area 6cm x 3cm were marked and missing cells were noted. The miniature colony was fed treated syrup, which was made by dissolving BPQ **B** powder in solvent, followed by serial dilutions using a sucrose solution (2 parts sugar: 1 part water). The concentrations administered are given below. Two replicates were carried out for the product, while 1 colony (apidae) was used for each of the solvent concentrations.

The following were the dilutions used:

<u>BPQ **B** in sucrose</u>

0.5μg/mL. (C1-C2)
0.05μg/mL. (D1-D2)
0.005μg/mL. (E1-E2)
0.0005μg/mL. (F1-F2)
0.00005μg/mL (G1-G2)

<u>Solvent in sugar:water feed</u> (equivalent solvent concentration to that in BPQ **B** -sugar water solutions)

0.001% (C4)
0.0001%. (D4)

0.00001%. (E4)
0.000001%. (F4)
0.0000001 (G4)

Results

Treating developing larvae with BPQ showed no negative effects on their development and the queen continued to lay during the treatment period. In the data below, the percentage emergence in the solvent treated colonies was lower than BPQ **B,** but the number of missing cells in this group was also higher, thus indicating a possible problem with the fertility of the eggs in these colonies.

Table 2

| **Feed 16_7_10** | | | | | |
|---|---|---|---|---|---|
| **Product** | **Totalcells** | **Missing cells** | **Sealed cells** | **Young bees emerged** | **% Emergence** |
| c1 | 40 | 5 | 32 | 30 | 85.7 |
| c2 | 40 | 7 | 33 | 31 | 93.9 |
| d1 | 40 | 7 | 30 | 27 | 81.8 |
| d2 | 40 | 7 | 29 | 23 | 69.7 |
| e1 | 40 | 6 | 31 | 28 | 82.4 |
| e2 | 40 | 3 | 37 | 32 | 86.5 |
| f1 | 40 | 3 | 32 | 26 | 70.3 |
| f2 | 40 | 8 | 30 | 28 | 87.5 |
| g1 | 40 | 9 | 30 | 30 | 96.8 |
| g2 | n/a | n/a | | | |
| **Solvent** | | | | | |
| c4 | 40 | 11 | 25 | 20 | 69.0 |
| d4 | 40 | 3 | 29 | 23 | 62.2 |
| e4 | 40 | 10 | 26 | 20 | 66.7 |
| f4 | 40 | 13 | 23 | 21 | 77.8 |
| g4 | 40 | 3 | 35 | 32 | 86.5 |
| **Control** | | | | | |
| x1 | 40 | 3 | 36 | 35 | 94.6 |
| x2 | 40 | 2 | 34 | 32 | 84.2 |

**Example 5 - Toxicity of BPQ A to *Varroa* mites**

(50mL liquid at a concentration of 50mg/mL - BPQ B)

Aim

To assess the toxicity of BPQ to the *Varroa* mite

Materials and methods

*Varroa* mites only feed on haemolymph of larvae and adult bees and thus would not ingest BPQ in a syrup/water solution. Therefore, it was necessary to identify a volume sufficient to permeate through the cuticle, but not drown the mites. After preliminary investigation it was concluded that 3μL/10 mites/eppendorf tube gave the maximum viability and the latter was used for this trial.

A total of 3μL of BPQ **A**/solvent was pipetted into each eppendorf tube. Ten mites taken from purple eye drone pupae were submerged in the liquid by tapping the tube gently and a fresh pupa was placed in each tube. 20 pinholes were made in each of the tubes and all samples were placed in the incubator at 30°C. Three replicates were made for each concentration and water was used in the control group. Concentrations of BPQ **A** and solvent were as described above (Section 1.1). The number of damaged or dead mites was counted on a daily basis. Damaged mites were mites that would not move in a coordinated fashion when touched gently 3 times with a fine brush.

Results

The survival curves indicate that at higher concentrations of BPQ, *Varroa* mite mortality is higher and more rapid than at lower concentrations (Fig. 9). At 0.5μg/mL (Fig. 9), the mean number of live mites on day 3 was 3.33±2.4 (mean±2.4) while at concentrations 0.0005μg/mL (f) and 0.00005μg/mL (g) the mean number of live mites on the same day was 7.66±1.33 and 8.66±1.73 respectively. It should be noted that not all mites were dead, but were damaged and immobile and consequently not able to stay attached to bees. This is typical of many treatments.

**Example 6 - Toxicity of BPQ B to *Varroa* mites on honeybees when fed treated sucrose solution**

(Powder version of BPQ, BPQ B, dissolved in the solvent (*N*-methyl pyrollidone)

Aim

To estimate the percentage efficacy of BPQ at different concentrations in sucrose solution

Materials and methods

A total of 20 newly emerged *Varroa* infested bees were placed in hoarding cages. The cages were prepared as described in Section 1.0, except that the air vents were reduced in size using parafilm to prevent the *Varroa* mites escaping during the test period. The mean(±se) number of mites per 20 bees was estimated prior to commencement of the experiment and an additional 5 mites per cage were added. (mean±se = 10.6±0.33). This was carried out by collecting adult female mite from drone pupae, placing them in an eppendorf tube and then releasing them into the cages.
The treatment solution was made by dissolving BPQ **B** in solvent, followed by two aqueous serial dilutions producing a concentration of 500μg/mL and 5 μg/mL. From the latter, further serial dilutions were carried with sucrose solution producing the following concentrations:

- 0.5μg/mL. (C1-C3)
- 0.05μg/mL. (D1-D3)
- 0.005μg/mL. (E1-E3)
- 0.0005μg/mL. (F1-F3)
- 0.00005μg/mL (G1-G3)

Three replicates were established at each concentration plus a control group which were only fed sugar syrup. All cages were placed in the incubator at 32°C and monitored on a daily basis for the number of mites and number of bees knocked down. At the end of five day treatment period, the percentage efficacy of the treatment was estimated by treating bees in all cages with Apistan (Trade Mark of Vita (Europe) Ltd. Apistan contains fluvalinate which is a synthetic pyrethroid.

$$\%\ \text{efficacy} = \text{mites knocked from treatment/ total mites (treatment+Apistan)} * 100$$

Results

The maximum efficacy of BPQ **B** fed in a sucrose solution was approximately 82% in treatment *f*(0.0005μg/mL), while the minimum was 43.8%, in treatment **g** (0.00005μg/mL). The latter was slightly higher than the percentage efficacy estimated for the control group (45.2%). A high proportion of mites were knocked down on day 2 (Table 3). However it should be noted that there was very high variation between replicates in each treatment concentration as shown in Fig. 11. Treatments had no negative effects on bee survival as indicated by Fig. 12. This is a remarkable efficacy result.

Table 3

| The daily *Varroa* mite drop down over the 5 day treatment period | | | | | | |
|---|---|---|---|---|---|---|
| **Treatment** | **Day 1** | **Day 2** | **Day 3** | **Day 4** | **Day 5** | **Day 6** |
| **c1** | 5+ | 8 | 1 | 1 | 2 | 1 |
| **c2** | 5+ | 2 | 2 | 1 | 0 | 1 |
| **c3** | 5+ | 1 | 0 | 0 | 1 | 2 |
| **d1** | 5+ | 6 | 2 | 3 | 1 | 2 |
| **d2** | 5+ | 10 | 0 | 0 | 1 | 2 |
| **d3** | 5+ | 4 | 1 | 3 | 1 | 0 |
| **e1** | 5+ | 2 | 1 | 0 | 1 | 3 |
| **e2** | 5+ | 5 | 0 | 3 | 0 | 0 |
| **e3** | 5+ | 12 | 2 | 1 | 3 | 0 |
| **f1** | 5+ | 7 | 0 | 0 | 1 | 1 |
| **f2** | 5+ | 9 | 0 | 1 | 0 | 1 |
| **f3** | 5+ | 9 | 1 | 0 | 0 | 0 |
| **g1** | 5+ | 2 | 0 | 0 | 2 | 0 |
| **g2** | 5+ | 14 | 0 | 0 | 0 | 0 |
| **g3** | 5+ | 0 | 1 | 0 | 0 | 0 |
| **x1** | 5+ | 6 | 1 | 0 | 1 | 0 |
| **x2** | 5+ | 1 | 2 | 4 | 2 | 1 |
| **x3** | 5+ | 2 | 0 | 3 | 0 | 0 |

**Example 7 - <u>Toxicity of BPQ B to *Varroa* mites on honeybees when sprayed with an aqueous solution of the active ingredient</u>**

(Powder version of BPQ, BPQ **B,** dissolved in the solvent (N-methyl pyrollidone)

<u>Aim</u>

To estimate the percentage efficacy of BPQ **B** at different concentrations when sprayed as an aqueous solution onto adult bees

<u>Materials and methods</u>

A total of 20 newly emerged *Varroa* infested bees were placed in hoarding cages. The cages were prepared as described in Section 1.0, except that the air vents were reduced in size using parafilm to prevent the *Varroa* mites escaping during the test period. The mean(±se) number of mites per 20 bees was estimated prior to commencement and an additional 10 mites per cage were added. Mites were collected and introduced as described above. The treatment solution was made by dissolving BPQ **B** in solvent, followed by two aqueous serial dilutions producing a concentration of 500μg/mL and 5μg/mL. From the latter, further serial dilutions were carried in water producing the following concentrations:

- 0.5μg/mL. (C1-C3)
- 0.05μg/mL. (D1-D3)
- 0.005μg/mL. (E1-E3)
- 0.0005μg/mL. (F1-F3)
- 0.00005μg/mL (G1-G3)

Three replicates were established at each concentration plus a control group which were sprayed with water only. All test groups were fed 10mL of 1:1 sucrose:water solution and placed in the incubator at 32°C and monitored on a daily basis for the number of mites and number of bees knocked down. At the end of five day treatment period, the percentage efficacy of the treatment was estimated by treating bees in all cages with Apistan.

% efficacy = mites knocked from treatment/ total mites (treatment+Apistan)*100

Results

Preliminary results indicate that BPQ **B** sprayed as an aqueous solution produced low percentage efficacy against the *Varroa* mite. The mean maximum efficacy was 46.2% (treatment g) and the mean minimum efficacy was 28.4% (treatment ***d***)(Fig. 13). However, there was high variation between the replicates (Fig. 14) at each concentration. The toxicity to bees was very low as indicated by the survival table (Table 4). The low toxicity is an encouraging result.

Table 4

| Survival rate on bees sprayed with different aqueous solutions of BPQ | | | | | | | |
|---|---|---|---|---|---|---|---|
| **Treatment** | **Day 1** | **Day 2** | **Day 3** | **Day 4** | **Day 5** | **Day 6** | **Bees remaining** |
| **c1** | 20 | 20 | 20 | 19 | 19 | 19 | 19 |
| **c2** | 20 | 20 | 20 | 20 | 20 | 20 | 20 |
| **c3** | 20 | 19 | 19 | 19 | 19 | 19 | 19 |
| **d1** | 20 | 20 | 20 | 20 | 20 | 20 | 20 |
| **d2** | 20 | 20 | 20 | 20 | 20 | 20 | 20 |
| **d3** | 20 | 19 | 19 | 19 | 19 | 19 | 19 |
| **e1** | 20 | 20 | 20 | 20 | 20 | 20 | 20 |
| **e2** | 20 | 20 | 19 | 19 | 19 | 19 | 19 |
| **e3** | 20 | 20 | 19 | 19 | 19 | 19 | 19 |
| **f1** | 20 | 20 | 20 | 20 | 20 | 20 | 20 |
| **f2** | 20 | 19 | 19 | 19 | 19 | 19 | 19 |
| **f3** | 20 | 18 | 18 | 18 | 18 | 18 | 18 |
| **g1** | 20 | 19 | 19 | 19 | 19 | 18 | 18 |
| **g2** | 20 | 19 | 17 | 17 | 17 | 17 | 17 |
| **g3** | 20 | 20 | 20 | 20 | 20 | 20 | 20 |
| **x1** | 20 | 17 | 16 | 16 | 16 | 16 | 16 |
| **x2** | 20 | 20 | 19 | 19 | 19 | 19 | 19 |
| **x3** | 20 | 20 | 20 | 20 | 20 | 20 | 20 |

- BPQ appears to be harmless to adult bees and larvae
- The product is highly toxic to free living *Varroa* mites
- *Varroa* mites attached to bees can be attacked via a bee feed route or through topical application
- Topical application is ~40% effective
- Application via bee feed route is 82% effective
- Mites detached through treatment with BPQ may be incapable of reattachment in contrast to mites detached in the caged control study

The invention is not limited to the embodiments hereinbefore described which may be varied in detail.

## Claims

1. Buparvaquone for use in controlling mites parasitising honeybees, which use comprises administering to the honeybees a non-toxic miticidally effective amount of burparvaquone.

2. Use as claimed in claim 1 wherein said administering comprises feeding buparvaquone to the bees.

3. Use as claimed in claim 1 or 2 wherein said administering comprises feeding buparvaquone in a sugar solution to the bees.

**4.** Use as claimed in any of claims 1 to 3 wherein the mites are *Varroa* mites.

**5.** A composition comprising a diet supplement for bees which contains a non-toxic miticidally effective amount of buparvaquone.

**6.** A composition as claimed in claim 5 wherein the diet supplement comprises a sugar solution.

**7.** A composition as claimed in claim 5 or 6 comprising a carrier for buparvaquone.

**8.** A composition as claimed in claim 7 wherein the carrier comprises a solvent.

**9.** A composition as claimed in claim 8 wherein the solvent comprises N-methyl-pyrollidone (NMP).

## Patentansprüche

**1.** Buparvaquon zur Verwendung bei der Kontrolle von Milben, die Honigbienen parasitieren, welche Verwendung das Verabreichen einer nichttoxischen, milbentötend wirksamen Menge von Buparvaquon an die Honigbienen umfasst.

**2.** Verwendung nach Anspruch 1, worin das genannte Verabreichen das Verfüttern von Buparvaquon an die Bienen umfasst.

**3.** Verwendung nach Anspruch 1 oder 2, worin das genannte Verabreichen das Verfüttern von Buparvaquon in einer Zuckerlösung an die Bienen umfasst.

**4.** Verwendung nach einem der Ansprüche 1 bis 3, worin die Milben *Varroa*-Milben sind.

**5.** Zusammensetzung, die ein Nahrungsergänzungsmittel für Bienen umfasst, das eine nichttoxische, milbentötend wirksame Menge von Buparvaquon enthält.

**6.** Zusammensetzung nach Anspruch 5, worin das Nahrungsergänzungsmittel eine Zuckerlösung umfasst.

**7.** Zusammensetzung nach Anspruch 5 oder 6, die einen Träger für Buparvaquon umfasst.

**8.** Zusammensetzung nach Anspruch 7, worin der Träger ein Lösungsmittel umfasst.

**9.** Zusammensetzung nach Anspruch 8, worin das Lösungsmittel N-Methylpyrrolidon (NMP) umfasst.

## Revendications

**1.** Buparvaquone en vue d’une utilisation dans le contrôle d’acariens parasitant les abeilles mellifères, ladite utilisation comprenant l’administration aux abeilles mellifères d’une quantité de buparvaquone non toxique à effet acaricide.

**2.** Utilisation selon la revendication 1, dans laquelle ladite administration comprend l’alimentation des abeilles avec de la buparvaquone.

**3.** Utilisation selon la revendication 1 ou 2, dans laquelle ladite administration comprend l’alimentation des abeilles avec de la buparvaquone dans une solution sucrée.

**4.** Utilisation selon l’une quelconque des revendications 1 à 3, dans laquelle les acariens sont des acariens du genre *Varroa.*

**5.** Composition comprenant un complément alimentaire destiné à des abeilles qui contient une quantité de buparvaquone non toxique à effet acaricide.

**6.** Composition selon la revendication 5, dans laquelle le complément alimentaire comprend une solution sucrée.

**7.** Composition selon la revendication 5 ou 6, qui comprend un véhicule pour la buparvaquone.

**8.** Composition selon la revendication 7, dans laquelle le véhicule comprend un solvant.

**9.** Composition selon la revendication 8, dans laquelle le solvant comprend du N-méthyl-pyrollidone (NMP).

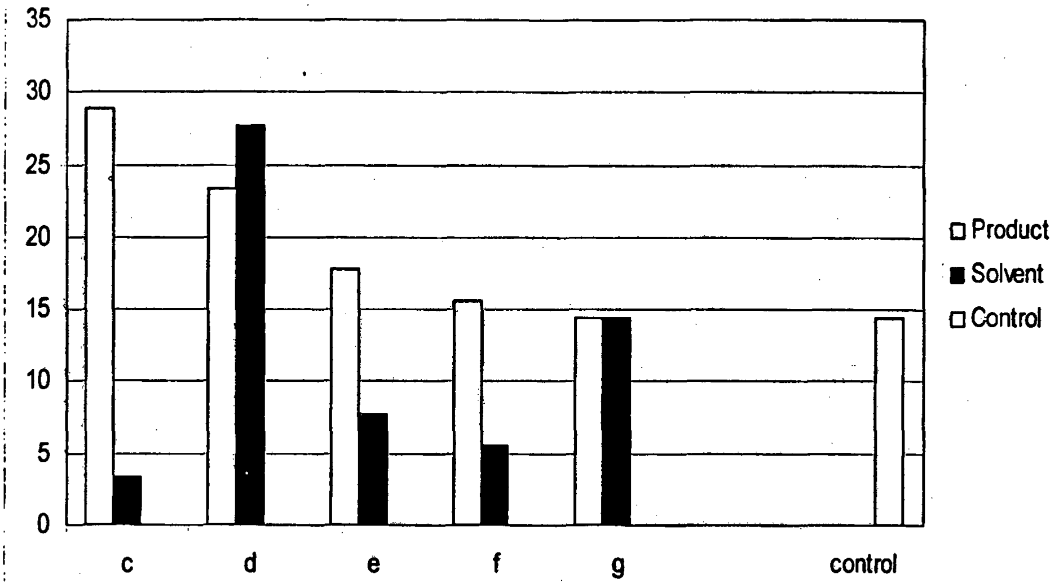
35
30
25
20
15
10
5
0
c
d
e
f
g
control
Product
Solvent
Control

Fig. 1

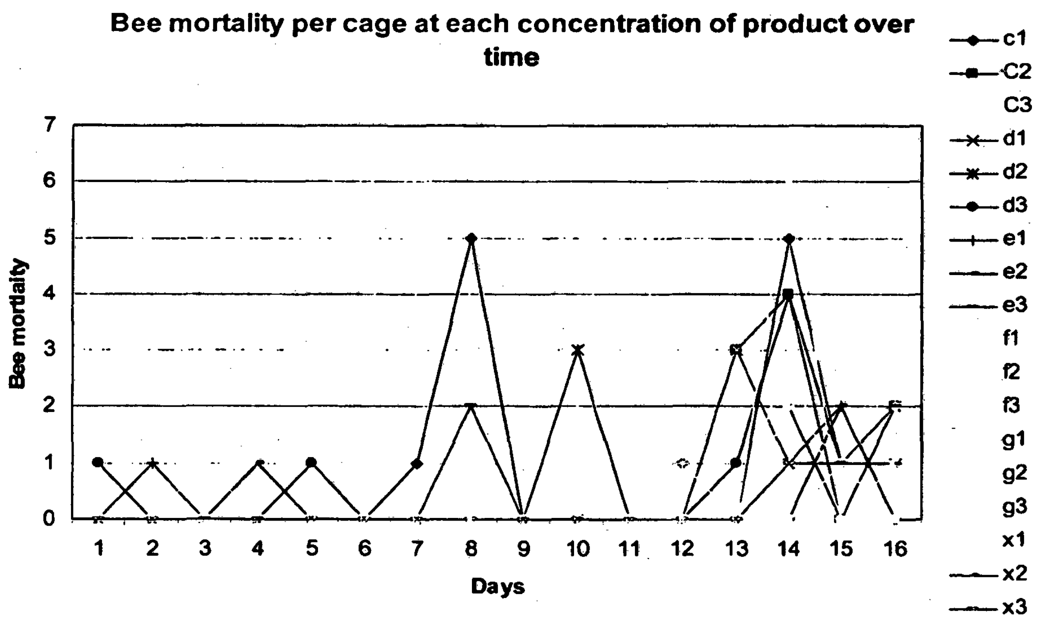
Bee mortality per cage at each concentration of product over time
Bee mortality
Days
c1
C2
C3
d1
d2
d3
e1
e2
e3
f1
f2
f3
g1
g2
g3
x1
x2
x3

Fig. 2

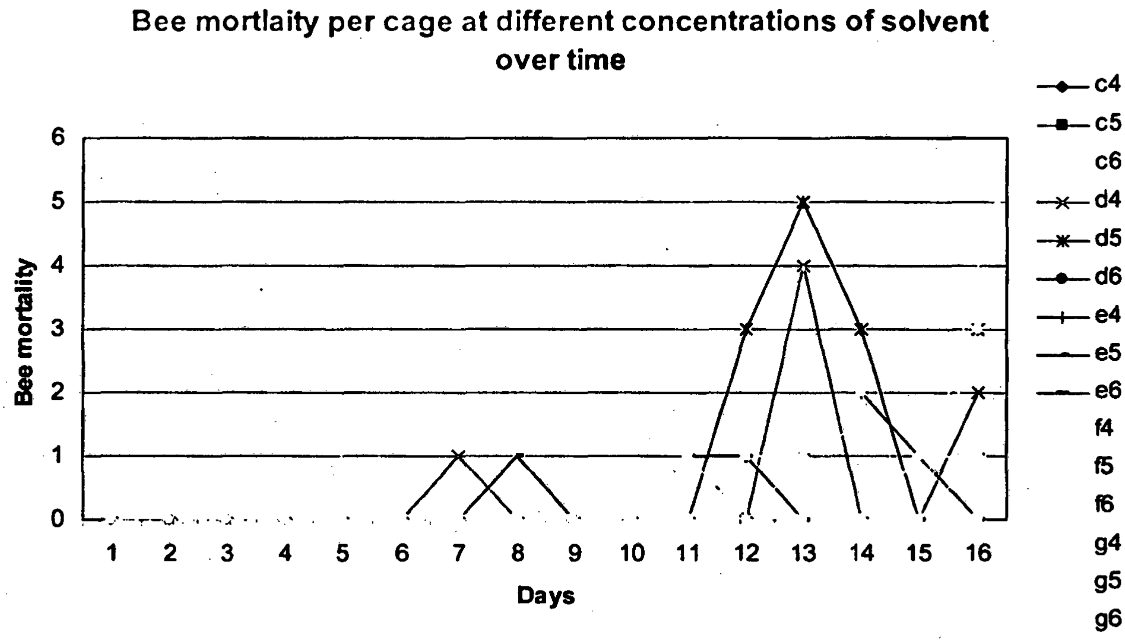
Bee mortlaity per cage at different concentrations of solvent over time
Bee mortality
0
1
2
3
4
5
6
1 2 3 4 5 6 7 8 9 10 11 12 13 14 15 16
Days
c4
c5
c6
d4
d5
d6
e4
e5
e6
f4
f5
f6
g4
g5
g6

Fig. 3

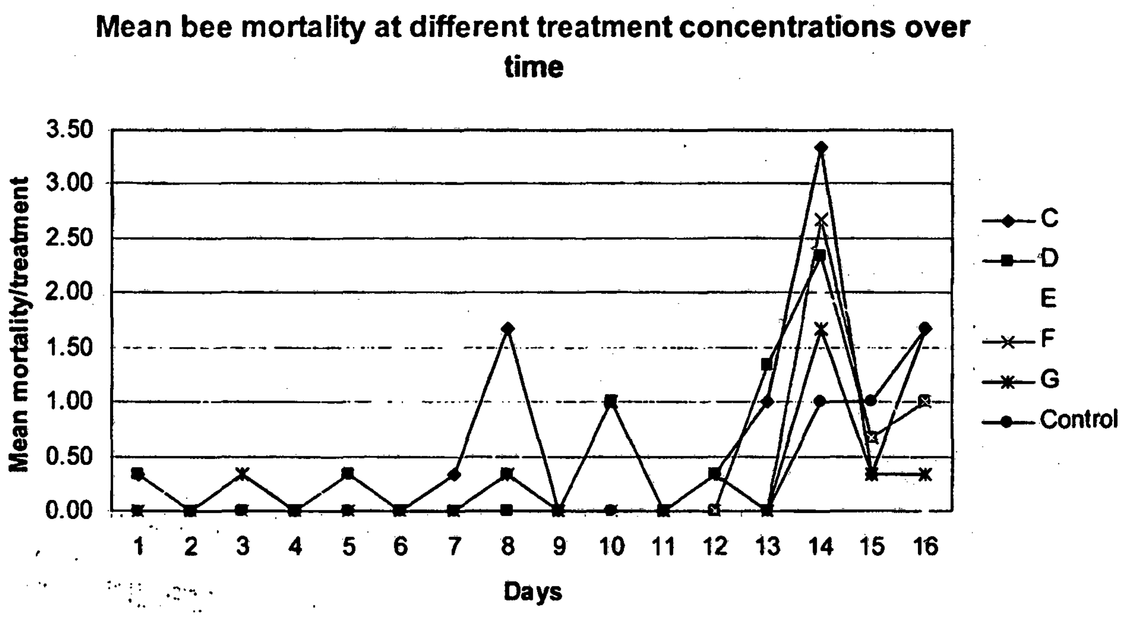
Mean bee mortality at different treatment concentrations over time
Mean mortality/treatment
0.00
0.50
1.00
1.50
2.00
2.50
3.00
3.50
1 2 3 4 5 6 7 8 9 10 11 12 13 14 15 16
Days
C
D
E
F
G
Control

Fig. 4

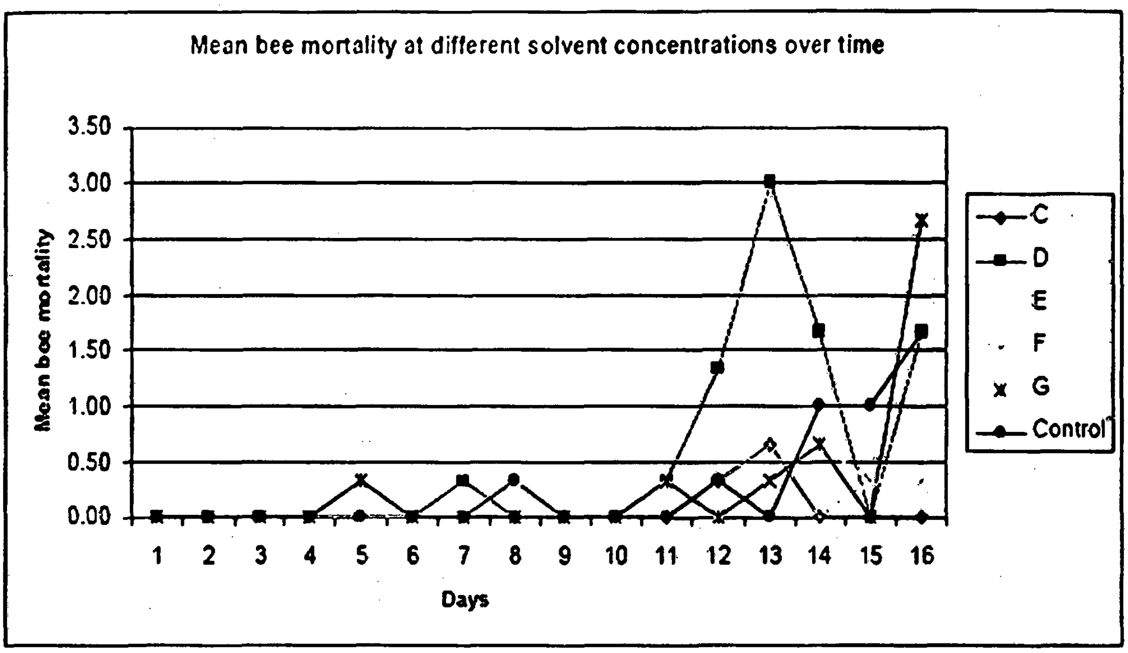

Mean bee mortality at different solvent concentrations over time
Mean bee mortality
3.50
3.00
2.50
2.00
1.50
1.00
0.50
0.00
1 2 3 4 5 6 7 8 9 10 11 12 13 14 15 16
Days
C
D
E
F
G
Control


Fig. 5

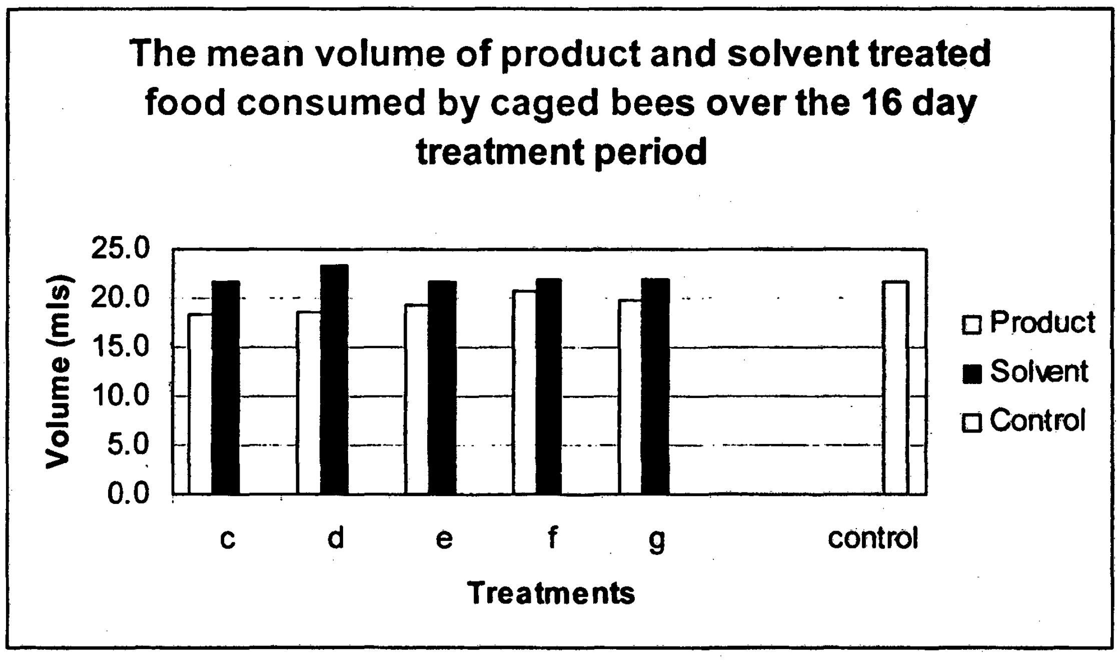

The mean volume of product and solvent treated food consumed by caged bees over the 16 day treatment period
Volume (mls)
25.0
20.0
15.0
10.0
5.0
0.0
c d e f g control
Treatments
Product
Solvent
Control


Fig. 6

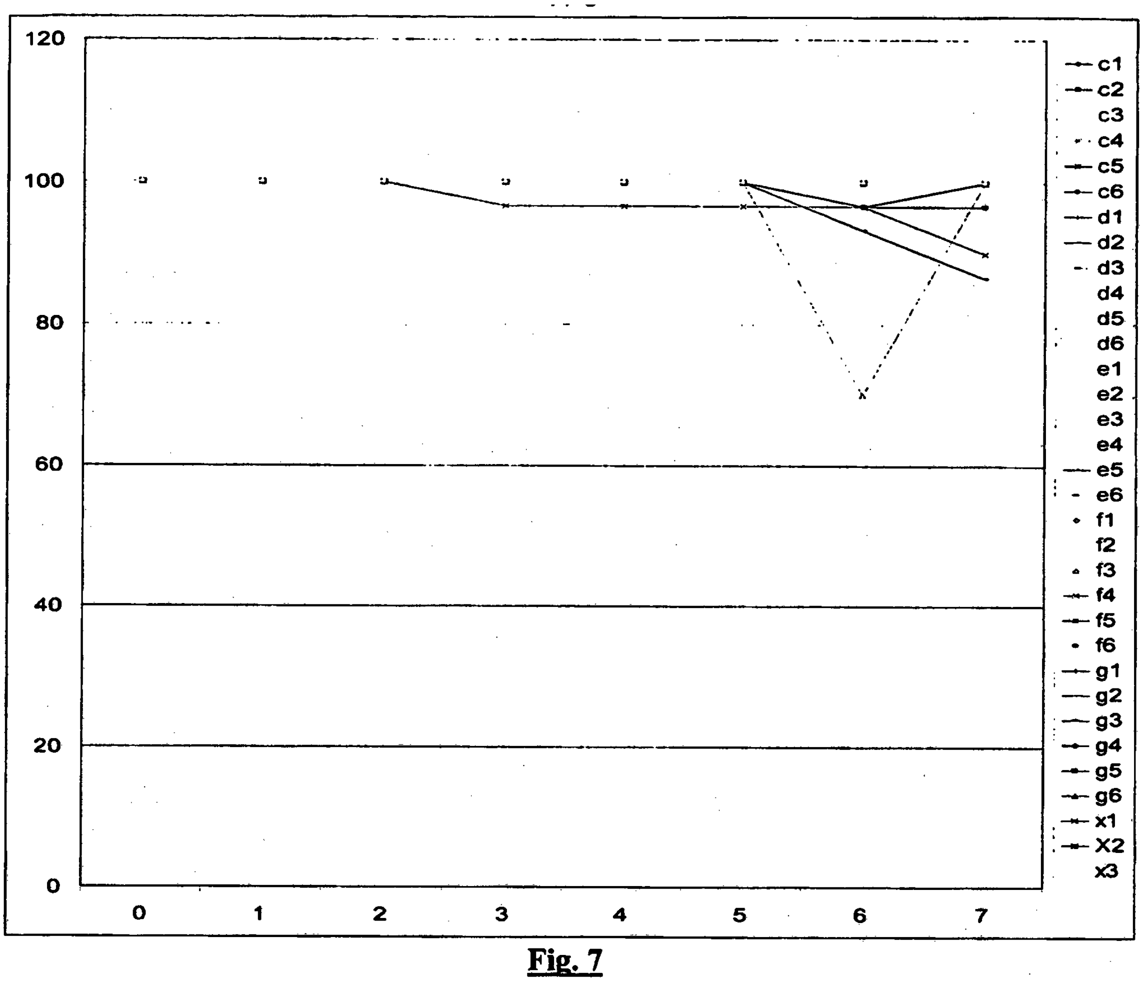
120
100
80
60
40
20
0
0
1
2
3
4
5
6
7
c1
c2
c3
c4
c5
c6
d1
d2
d3
d4
d5
d6
e1
e2
e3
e4
e5
e6
f1
f2
f3
f4
f5
f6
g1
g2
g3
g4
g5
g6
x1
X2
x3

Fig. 7

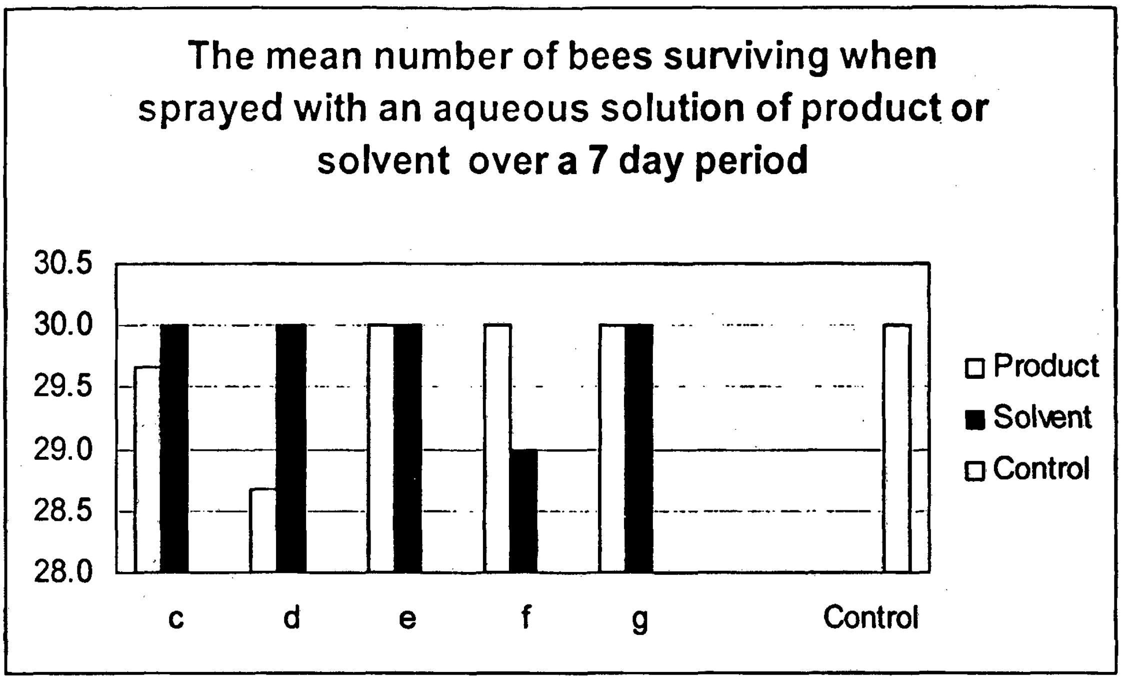
The mean number of bees surviving when sprayed with an aqueous solution of product or solvent over a 7 day period
30.5
30.0
29.5
29.0
28.5
28.0
c
d
e
f
g
Control
Product
Solvent
Control

Fig. 8

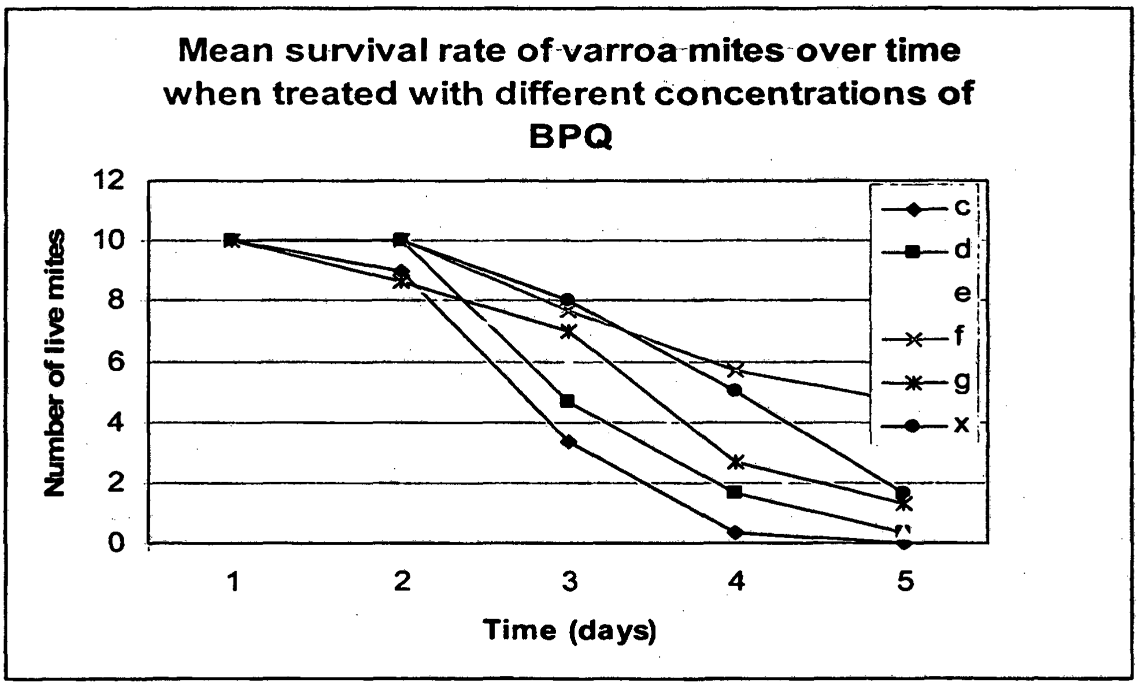
Mean survival rate of varroa mites over time when treated with different concentrations of BPQ
Number of live mites
12
10
8
6
4
2
0
1
2
3
4
5
Time (days)
c
d
e
f
g
x

Fig. 9

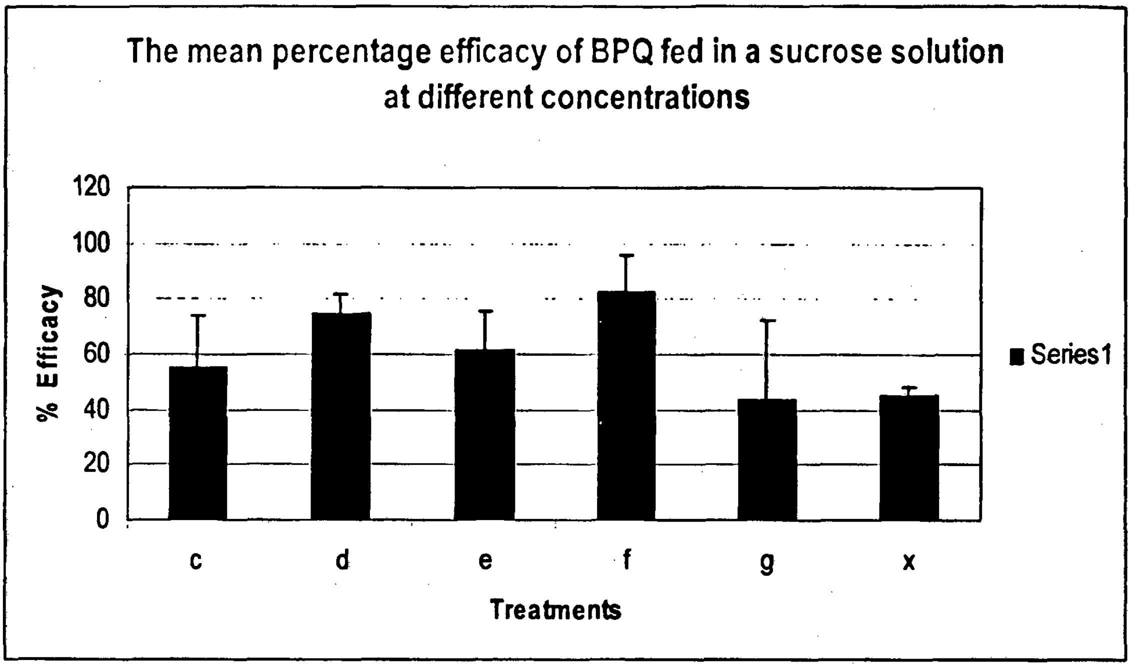
The mean percentage efficacy of BPQ fed in a sucrose solution at different concentrations
120
100
80
60
40
20
0
% Efficacy
c
d
e
f
g
x
Treatments
Series1

Fig. 10

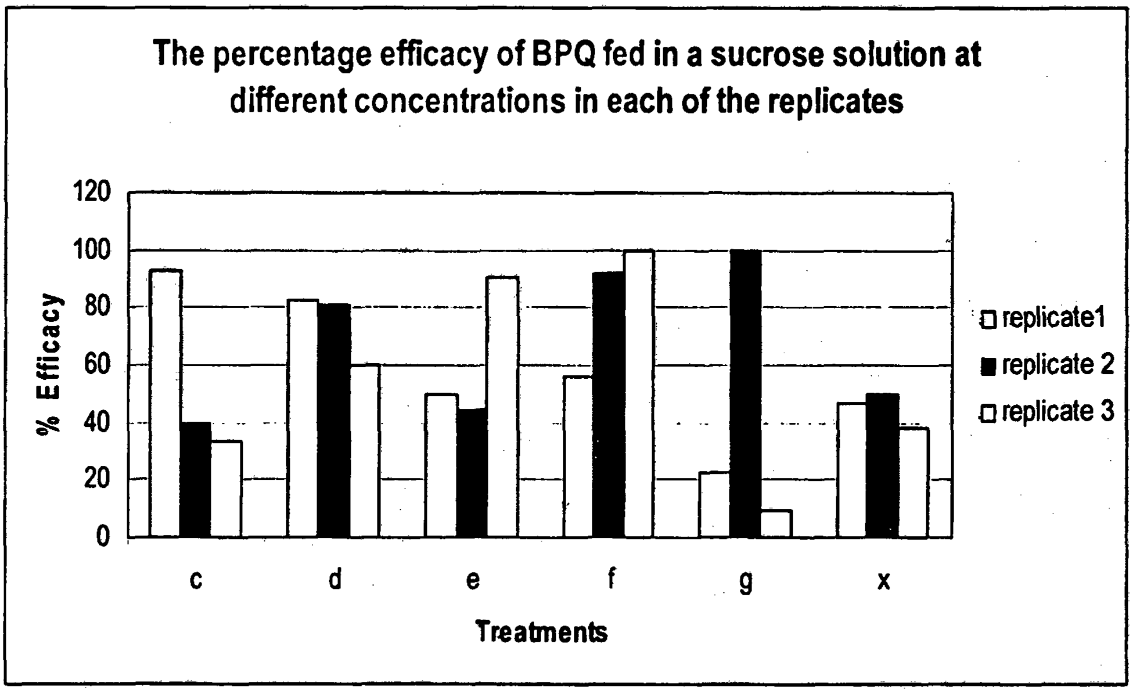
The percentage efficacy of BPQ fed in a sucrose solution at different concentrations in each of the replicates
120
100
80
60
40
20
0
% Efficacy
c
d
e
f
g
x
Treatments
replicate1
replicate 2
replicate 3

Fig. 11

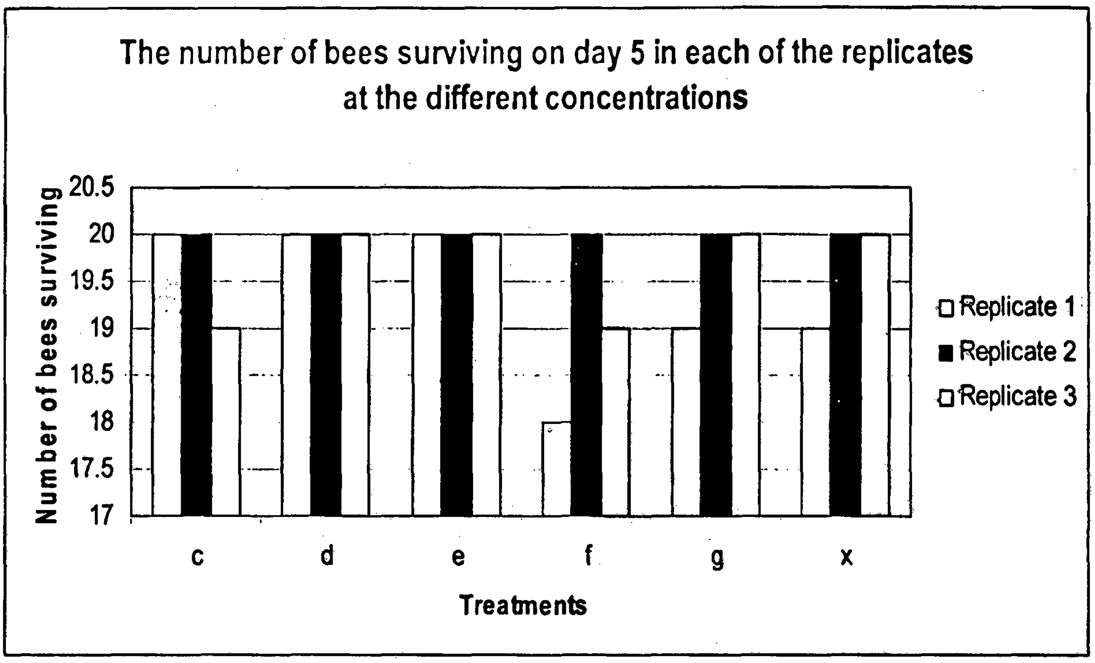
The number of bees surviving on day 5 in each of the replicates at the different concentrations
Number of bees surviving
20.5
20
19.5
19
18.5
18
17.5
17
c
d
e
f
g
x
Treatments
Replicate 1
Replicate 2
Replicate 3

**Fig. 12**

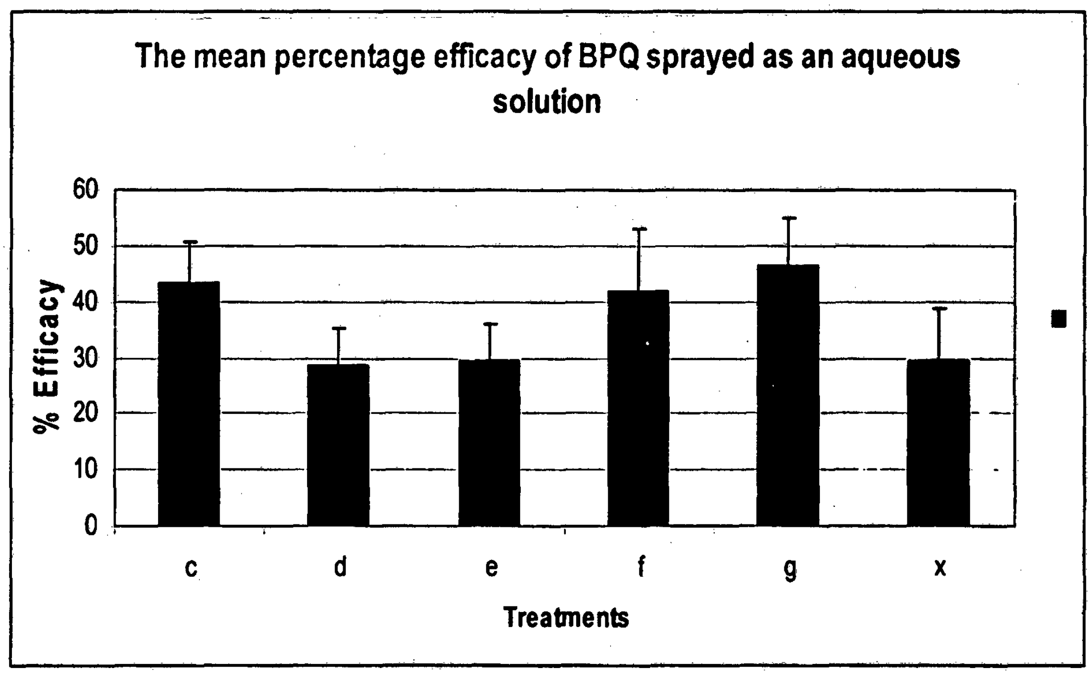
The mean percentage efficacy of BPQ sprayed as an aqueous solution
% Efficacy
60
50
40
30
20
10
0
c
d
e
f
g
x
Treatments

**Fig. 13**

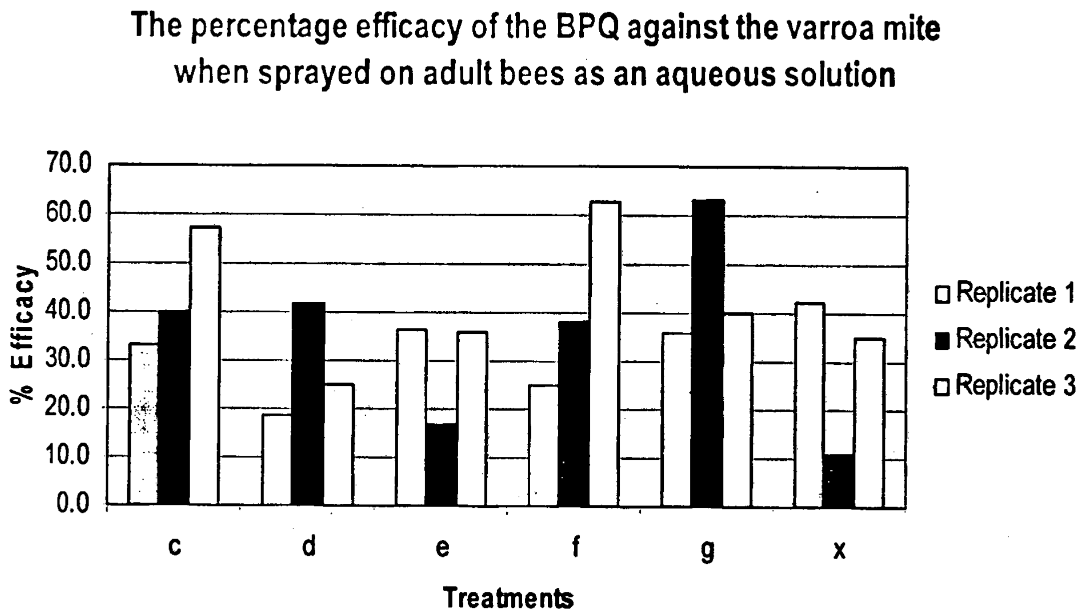
The percentage efficacy of the BPQ against the varroa mite
when sprayed on adult bees as an aqueous solution
% Efficacy
70.0
60.0
50.0
40.0
30.0
20.0
10.0
0.0
c
d
e
f
g
x
Treatments
Replicate 1
Replicate 2
Replicate 3

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MBWAMBO et al.** *Veterinary Parasitology,* 2006, vol. 139 (1-3), 67-73
- **KAYSER et al.** *Parasitology Research,* 2003, vol. 90 (2), s63-s70
- **KAYSER.** *International Journal of Pharmaceutics,* 2001, vol. 214, 83-85